# EUROPEAN PATENT APPLICATION

(11) **EP 3 470 103 A1**
(43) Date of publication of application: **17.04.2019**
(21) Application number: 17001665.3
(22) Date of filing: 10.10.2017
(51) Int. Cl.: A61M 16/08, A61M 16/06, A61M 16/00

(54) **ELBOW CONNECTOR FOR RESPIRATORY MASK WITH A MESH STRUCTURE FOR VENTING GAS EXPIRED BY THE PATIENT**

(71) Applicant: Air Liquide Medical Systems, 92160 Antony (FR)
(72) Inventor: Alberici, Luca, 25030 Roncadelle (BS) (IT); Masserdotti, Fulvio, 25075 Nave (IT); Massaro, Paolo, 25127 Brescia (BS) (IT)
(74) Representative: Pittis, Olivier

(57) **Abstract**

The invention concerns an elbow connector (1) comprising a hollow elbow-shape body (7) comprising an inner gas path (4) having an inlet orifice (2) and an outlet orifice (3), and a large port (5) traversing said elbow-shape body (7) so as to put the inner gas path (4) in fluid communication with the atmosphere. The large port (5) is covered by a mesh structure (6) comprising polymer fibers (8). Respiratory mask (20) comprising such an elbow connector (1)

## Description

The present invention concerns an elbow-shape connector comprising an improved venting system for venting the CO₂-enriched gas expired by a patient to the atmosphere and a respiratory mask, preferably a nasal mask, suitable for treating sleep apnea or other respiratory diseases, comprising such an elbow-shape connector.

The respiratory masks that are used for treating sleep apnea are called "sleep apnea masks". A sleep apnea mask can be either a nasal or full face mask, comprising one or several exhalation orifices, also called venting or exhalation holes, ensuring the washout of the enriched-CO₂ gas expired by the patient. Venting holes are arranged either in the mask body as disclosed by EP-A-1582230, or in the tubular connector, typically an elbow-connector, used for fluidly connecting a flexible hose, or a similar fluid conduit, to the mask body as described by EP-A-2281597. Similar mask arrangements are also taught by WO-A-2005/051468 and EP-A-462701.

However, existing connectors equipping respiratory masks are not totally satisfactory and need to be improved because:
- they do not ensure an efficient venting of the CO₂-enriched gases through their venting holes, and/or
- they are too noisy due to the "whistling" emitted by the passage of the gas through the holes of the connector, while the patient is expiring gas.

Hence, the goal of the present invention is to provide an improved elbow-connector suitable for respiratory masks, especially masks used for treating sleep apnea or other respiratory diseases.

The solution of the present invention is an elbow connector for a respiratory mask comprising:
- a hollow elbow-shape body comprising an inner gas path having an inlet orifice and an outlet orifice, and
- a large port traversing said elbow-shape body so as to put the inner gas path in fluid communication with the atmosphere,
characterized in that the large port is covered by a mesh structure comprising polymer fibers.

The elbow connector according to the present invention can further comprise one or more of the following additional features:
- the elbow-shape body is made of plastic material, such as polycarbonate.
- the elbow-shape body is tubular, preferably having a circular cross-section.
- the polymer fibers are made of polyester (e.g., polyethylene terephthalate or PET) or polyamide (i.e., PA).
- the polymer fibers have a diameter of between 25 and 50 µm.
- the mesh structure comprises meshes having a square shape.
- the mesh structure comprises square-shape meshes having a dimension of between 20 and 200 µm.
- the mesh structure is secured by a fixation frame or directly welded to the elbow shaped body.
- the fixation frame has an annular shape.
- the fixation frame is fixed to the elbow-shape body.
- the fixation frame is detachably attached to the elbow-shape body, for instance plugged, press-fitted or the like.
- alternatively, the fixation frame is glued or ultrasonically welded to the elbow-shape body.
- the fixation frame is made of polymer material.
- the fixation frame comprises one or several ribs.
- the large port traversing the elbow-shape body has a size of between 5 and 30 mm.

The invention also concerns a respiratory mask comprising an elbow connector according to the invention, preferably a nasal mask, typically used for treating sleep apnea or other respiratory diseases. The respiratory mask can also be another kind of mask, such as a facial mask or a nasal pillow mask.

The respiratory mask comprises a shell, also called mask body, and a flexible cushion. The shell and the cushion can be made of several sub-units attached together or formed in one-piece. The elbow connector according to the invention is fluidly connected to the shell.

Further, the cushion comprises a central aperture, i.e. a large opening, for receiving the patient's nose, also called "nose opening". It is made of soft, resilient, elastomeric material, such as silicone, that comes into contact with the patient's face.

The shell and the flexible cushion defines an internal breathing chamber or volume, wherein respiratory gas, such as air under pressure, is introduced via an inlet port arranged at the center of the mask body, which is fluidly connected to a gas feeding line, such as a flexible gas conduit, by means of the elbow connector assembly according to the present invention, thereby allowing air to be introduced in the breathing chamber that receives the nose of the patient.

The mask body or shell is preferably made of a polymer material, such as polycarbonate (PC), polypropylene (PP), ABS, nylon or polystyrene (PS).

In use, the patient introduces his/her nose through the large opening of the cushion and into the internal volume of the breathing chamber that contains gas, and breathes some gas contained therein.

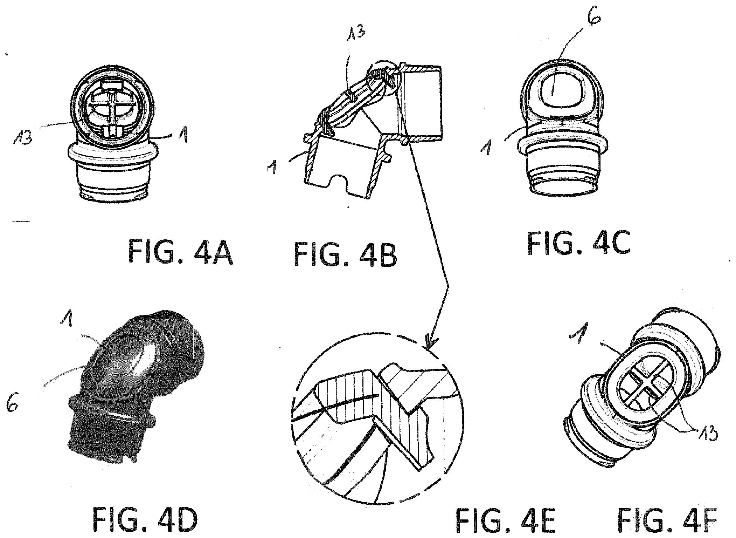

The shell preferably has a general triangular or quasi-triangular shape as shown in Figure 4. Preferably, the shell of the mask further comprises:
- two lateral arms projecting on each side, i.e. left and right sides, of the shell, and
- an upwardly-projecting arm arranged on the top of the shell and projecting upwardly from said shell.

The lateral and the upwardly-projecting arms comprise, at their free ends, headgear fixation structures, like hooks, slots or the like, for fixing a headgear thereto, in particular straps made of fabric, polymer material or the like. The headgear is used for fixing and maintaining the mask on the face of the user, i.e. the patient.

Furthermore, the invention also concerns a respiratory gas delivering assembly comprising:
- a gas source, preferably a CPAP or BPAP device, delivering a pressurized respiratory gas, such as air under pressure (i.e. pressure > 1 atm), and
- a respiratory mask according to the present invention, preferably a nasal mask that covers the nose of the patient

Further, a flexible gas line, such as a plastic hose or the like, is used for conveying the gas delivered by the gas source to the elbow connector of the respiratory mask thereby delivering said gas to the airways of the patient Preferably, the flexible gas line is fluidly connected to the elbow connector by means of an intermediary rotatable connector that rotates around it axis with respect to the elbow connector.

Other features, aspects and advantages of the present invention will become apparent from the following detailed description when taken in conjunction with the accompanying drawings which illustrates, by way of examples, the present invention, among which:
- Figures 1A-1D represent a first embodiment of an elbow connector according to the present invention,
- Figures 2A-2D represent a second embodiment of an elbow connector according to the present invention,
- Figures 3A-3D represent a third embodiment of an elbow connector according to the present invention,
- Figures 4A-4F represent a fourth embodiment of an elbow connector according to the present invention,
- Figure 5 represents a respiratory mask comprising a connector according to the present invention, and
- Figure 6 represents an enlarged view of the mesh structure arranged on an elbow connector according to the present invention.

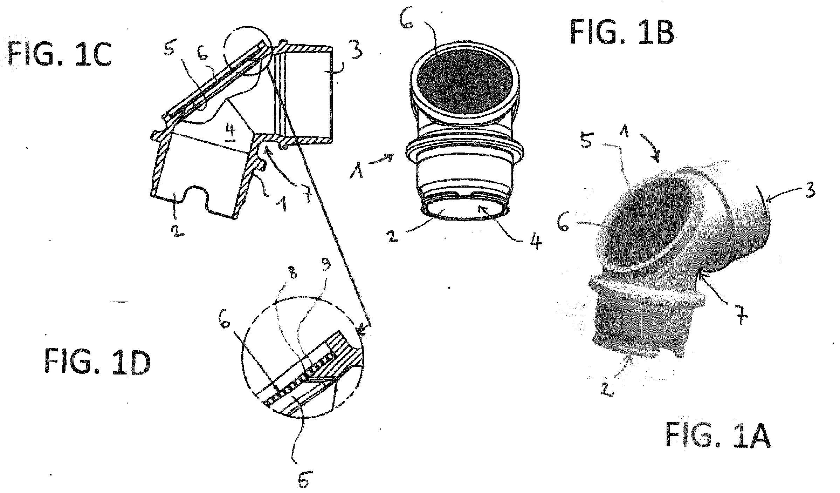

Figures 1A-1D represent a first embodiment of an elbow connector 1 for a respiratory mask according to the present invention.

The connector 1 comprises a hollow elbow-shape structure 7, namely a tubular bent structure, preferably made of plastic material, such as polycarbonate or similar, comprising an inner gas path 4, also called gas passage or lumen, for conveying a respiratory gas, such as air, to a mask 20 worn by a patient (see Fig. 4). The inner gas path 3 comprises an inlet orifice 2 allowing gas to enter into the lumen and an outlet orifice 3 allowing gas to leave the lumen and enter into the breathing chamber of the mask 20.

In other words, the pressurized respiratory gas normally circulates in the inner gas path 4 from the inlet orifice 2 to the outlet orifice 3 so as to be delivered afterwards to the respiratory mask 20, especially a nasal mask, and then to the patient's airways, during the inspiration phases of the patient

Further, the elbow-shape body 7 further comprises a large port 5, also called "aperture" or similar, traversing the wall of the elbow-shape body 7 so as to put the inner gas path 4 in fluid communication with the atmosphere, i.e. ambient air.

Said large port 5 is located in the elbow portion of the elbow connector 1 as shown in Fig. 1A and 1C. Large port 5 constitutes a venting passage, i.e. a gas exit, for venting to the atmosphere, the CO₂-containing gas expired by the patient, during his/her expiration phases.

Preferably, large port 5 has an oval or circular section as shown in Fig. 1B and/or has a size, such as a diameter, of between 5 mm and 30 mm.

According to the present invention, large port 5 is covered by a mesh structure 6 comprising polymer fibers 8, for example a polymer such as polyester, e.g. PET, or polyamide (PA). The polymer fibers 8 may have a diameter of between 25 and 50 µm.

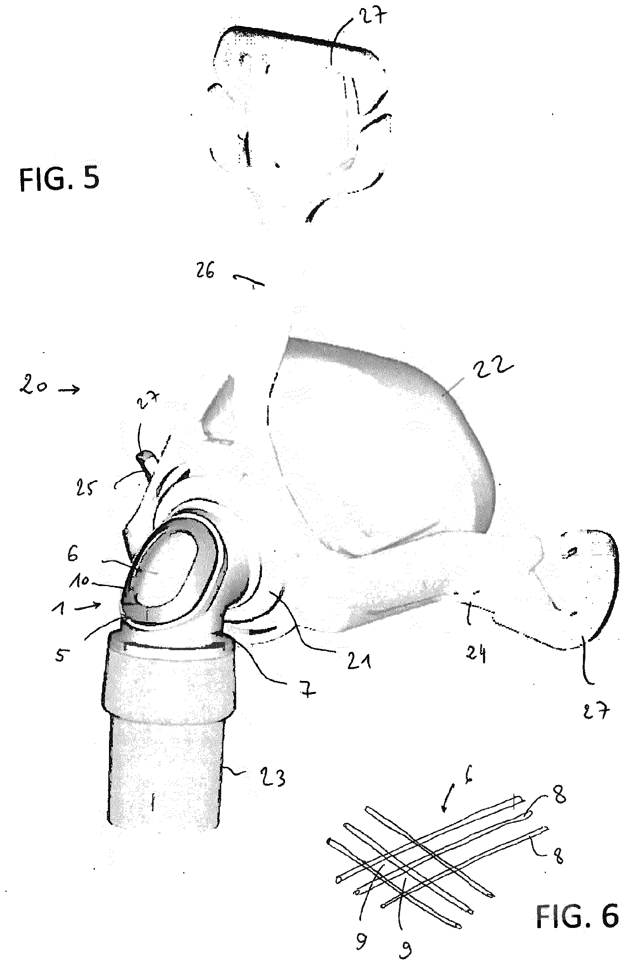

Further, as represented in Figure 6, the mesh structure 6 comprises a plurality of meshes 9 having each a square shape, preferably having a dimension of between 20 and 200 µm. The mesh structure 6 will normally constitute something similar to a fabric structure.

In a first embodiment, the mesh structure 6 can be glued, plugged-in or welded to the elbow-shape body 7, as shown in Fig. 1B.

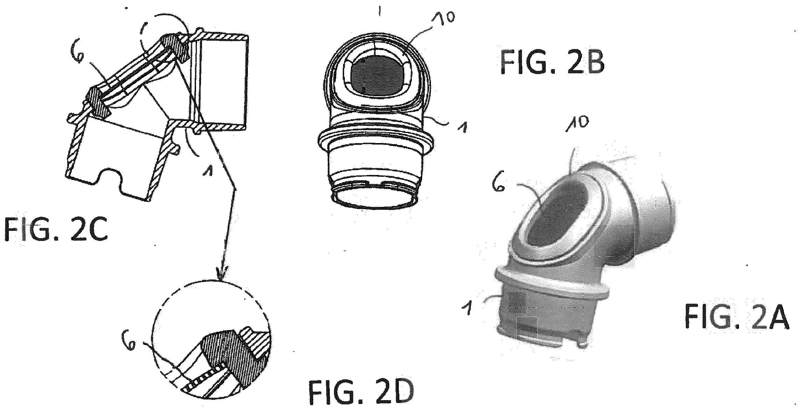

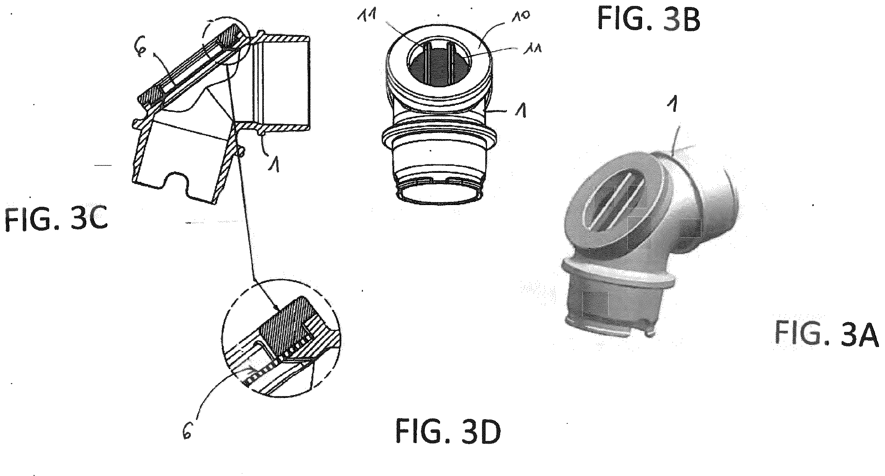

Figures 2A-2D represent a second embodiment of an elbow connector 1 according to the present invention that is similar to the first embodiment, except that the mesh structure 6 is secured to the elbow-shape body 7 by a fixation frame 10, preferably a fixation frame 10 having an annular shape as shown in Fig. 2B or 3B, and/or made of polymer material, such as polycarbonate (PC), polypropylene (PP), polyamide (PA).

Figures 3A-3D represent a third embodiment of an elbow connector 1 according to the present invention that is similar to the second embodiment, except that the fixation frame 10 securing the mesh structure 6 comprises two outer ribs 11 that are used for protecting the mesh structure 6, for instance for preventing that the mesh is accidently crushed from the outside.

Figures 4A-4F represent a fourth embodiment of an elbow connector 1 according to the present invention that is similar to the third embodiment, except that the fixation frame 10 securing the mesh structure 6 further comprises inner ribs 13 in lieu of outer ribs. In that embodiment, the inner ribs 13 are arranged crosswise, but they could be also arranged otherwise. The role of the inner ribs 13 is to hold the mesh structure 6 if it is accidently crushed down.

Generally speaking, the first end comprising the outlet orifice 3 of the elbow connector 1 of Fig. 1-4 is configured for being connected, in a known manner, e.g. plugged, to the shell 21 of a respiratory mask 20, such as a nasal mask sized for covering the nose of a user, i.e. a patient, as shown in Figure 5. However, it could be also used with other kinds of masks, such as facial or nasal pillow masks.

The mask 20 is held in position on the patient's face by means of a headgear comprising straps made of fabric, polymer or similar, that is attached to several arms 24, 25, 26 fixed to the shell 21 of the mask 20, namely two lateral arms 24, 25 extending on the right and left sides of the mask 20 and an upwardly-projecting arm 26. Those arms 24, 25, 26 comprise at their free ends, headgear connecting structures 27, such as hooks, slots or the like, for fixing a headgear thereto.

The mask 20 further comprises a flexible cushion 22, preferably made of silicone or the like, attached to the shell 21 and that contacts the patient's face, when the patient wears the mask 20. The cushion 22 comprises a large opening for receiving the nose of the patient and allowing him/her respiring gas into the respiratory chamber of the mask 20, that is delimited by the inner walls of the cushion 22 and/or shell 21, depending on the general configuration of the mask 20. The perimeter of said large opening is bordered by one or several thin membrane(s) ensuring a gas tightness with the patient's face.

Further, the second end of the elbow connector 1 comprising the inlet orifice 2 is configured for being connected, in a known manner, by means of an intermediary rotatable connector 23 to a flexible gas line, namely a plastic hose or similar fluid conduit, receiving pressurized gas delivered by a gas source, such as a CPAP or BPAP device or any other suitable gas delivery apparatus.

The mask 20 according to the present invention is useable for treating respiratory failures or disorders, such as sleep apnea or the like.

## Claims

1. Elbow connector (1) comprising:
- a hollow elbow-shape body (7) comprising an inner gas path (4) having an inlet orifice (2) and an outlet orifice (3), and
- a large port (5) traversing said elbow-shape body (7) so as to put the inner gas path (4) in fluid communication with the atmosphere,
**characterized in that** the large port (5) is covered by a mesh structure (6) comprising polymer fibers (8).

2. Elbow connector according to the previous Claim, **characterized in that** the polymer fibers (8) are made of polyester or polyamide.

3. Elbow connector according to any one of the previous Claims, **characterized in that** the polymer fibers (8) have a diameter of between 25 and 50 µm.

4. Elbow connector according to any one of the previous Claims, **characterized in that** the mesh structure (6) comprises meshes (9) having a square shape.

5. Elbow connector according to any one of the previous Claims, **characterized in that** the mesh structure (6) comprises square-shape meshes having a dimension of between 20 and 200 µm.

6. Elbow connector according to any one of the previous Claims, **characterized in that** the mesh structure (6) is secured by a fixation frame (10).

7. Elbow connector according to any one of the previous Claims, **characterized in that** the fixation frame (10) has an annular shape.

8. Elbow connector according to any one of the previous Claims, **characterized in that** the fixation frame (10) is fixed to the elbow-shape body (7).

9. Elbow connector according to any one of the previous Claims, **characterized in that** the fixation frame (10) is made of polymer material.

10. Elbow connector according to any one of the previous Claims, **characterized in that** the fixation frame (10) comprises one or several ribs (12, 13).

11. Elbow connector according to any one of the previous Claims, **characterized in that** the large port (5) has a size of between 5 and 30 mm.

12. Respiratory mask (20) comprising an elbow connector (1) according to any one of the previous claims, preferably a nasal mask.

13. Respiratory gas delivering assembly comprising a gas source, preferably a CPAP or BPAP device, delivering a pressurized respiratory gas, and a respiratory mask (20) according to Claim 12.

14. Respiratory gas delivering assembly according to Claim 13, **characterized in that** a flexible gas line is fluidly connected to the gas source and to elbow connector (1) for conveying the gas delivered by the gas source to the elbow connector (1) of the respiratory mask (20).

15. Respiratory gas delivering assembly according to Claim 13, **characterized in that** the flexible gas line is fluidly connected to the elbow connector (1) by means of an intermediary rotatable connector (23).
